# EUROPEAN PATENT SPECIFICATION

(11) **EP 3 563 856 B1**
(45) Date of publication and mention of the grant of the patent: **04.11.2020**
(21) Application number: 17888483.9
(22) Date of filing: 28.12.2017
(51) Int. Cl.: A61K 31/7068, A61K 31/4412, A61K 31/53, A61P 35/00

(54) **PHARMACEUTICAL COMPOSITION COMPRISING CAPECITABINE, GIMERACIL, AND OTERACIL FOR TREATING CANCER AND USE THEREOF**
PHARMAZEUTISCHE ZUSAMMENSETZUNG ENTALTEND CAPECITABINE, GIMERACIL UND OTERACIL ZUR BEHANDLUNG VON KREBS UND VERWENDUNG DAVON
COMPOSITION PHARMACEUTIQUE COMPRÉNANT CAPECITABINE, GIMERACIL ET OTERACIL DESTINÉE AU TRAITEMENT DU CANCER, ET UTILISATION CORRESPONDANTE

(30) Priority: 30.12.2016 CN 201611257037
(43) Date of publication of application: 06.11.2019
(73) Proprietor: Chen, Xiaohua, Nanjing, Jiangsu 210009 (CN)
(72) Inventor: Chen, Xiaohua, Nanjing, Jiangsu 210009 (CN)
(74) Representative: Huang, Liwei
(86) International application number: PCT/CN2017/119360
(87) International publication number: WO 2018/121669

(56) References cited:
- EP-A1- 2 716 290
- CN-A- 101 068 549
- CN-A- 101 909 602
- CN-A- 102 028 685
- CN-A- 104 434 925
- CN-A- 104 922 131
- CN-A- 105 726 567
- CN-A- 106 619 689
- MIWA M ET AL: "Design of a novel oral fluoropyrimidine carbamate, capecitabine, which generates 5-fluorouracil selectively in tumours by enzymes concentrated in human liver and cancer tissue", EUROPEAN JOURNAL OF CANCER, ELSEVIER, AMSTERDAM, NL, vol. 34, no. 8, 1 July 1998 (1998-07-01), pages 1274-1281, XP004285808, ISSN: 0959-8049, DOI: 10.1016/S0959-8049(98)00058-6

## Description

### BACKGROUND OF THE INVENTION

### Technical Field

The invention relates to a pharmaceutical composition for treating cancers, a kit, and the use thereof, belonging to the technical field of drugs.

### Description of Related Art

Cancers have become the main diseases that endanger the health of human beings. At present, the main treatment approach of cancers is chemotherapy. However, chemotherapeutics have been gradually changed from the traditional cytotoxic drugs into targeted molecular drugs with a targeting ability and high cancer cell selectivity. 5-Fluorouracil (5-FU) was one of the earliest anti-metabolism drugs widely used for treating cancers, and is an inhibitor of thymidylate synthas. 5-FU has a practical effect regarding anti-cancer treatment, but has a short in-vivo half-life period. During clinical use, 5-FU is usually continuously fed via veins, which generates many toxic and side effects, thus limiting the clinical applications thereof. In order to overcome the defects, science researchers developed a prodrug of 5-FU and compositions of other drugs to increase the therapeutic effect and reduce side effects.

### 1. Tegafur

Tegafur, with a chemical name of 1-(etrahydro-2-furyl)-5-fluoro-2,4(1H,3H)-pyrimidinedione, belongs to the pyrimidine anti-cancer drugs, is a prodrug of 5-FU, and has an inhibiting effect on the majority of solid cancers. Tegafur can interfere with and interdict the biological synthesis of DNA, RNA, and proteins, thus generating an anti-cancer effect. However, tegafur has serious side effects such as myelosuppression, gastrointestinal reactions, and lesions of the liver and kidney, and therefore is seldom applied to individual clinical use. Usually, tegafur needs to be used in combination with other drugs to reduce the serious side effects.

The structural formula of Tegafur is as follows:

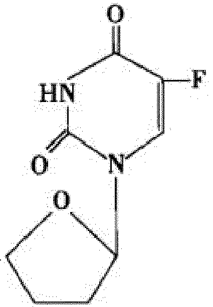

### 2. Capecitabine

Capecitabine, with a chemical name of 5'-deoxy-5-fluoro-N[(pentyloxy)carbonyl]cytidine, is a novel anti-cancer drug researched and manufactured by the Roche Group. This drug was approved to enter the American market by the FDA in 1998, with a trade name of XELODA. At present, the import of this product has been approved by China. Capecitabine clinically applies to the treatment of advanced metastatic breast cancer, colorectal cancer, and other solid cancers, and has a good anti-cancer effect and few adverse effects.

Capecitabine is a selective oral fluorouracil carbamate type anti-cancer drug with a huge potential, which is activated in the cancer and is converted into fluorouracil (FU) through three types of enzymes in the liver and in the cancer. After being taken orally, capecitabine as a whole piece passes through the gastrointestinal walls, is first catalyzed by carboxylesterase of the liver and metabolized into 5'-deoxy-5-fluorocytidine (5'-DFCR), then is catalyzed by the cytidine deaminase in the liver and cancer cells and converted into 5'-deoxy-5-fluorouracil (5'-DFUR), and finally

is catalyzed by the thymidine phosphorylase (TP) and converted into fluorouracil (FU), to play a cytotoxic effect. Capecitabine and tegafur are both prodrugs of 5-fluorouracil, but the high cancer selectivity and specificity of the capecitabine are obviously superior to those of the Tegafur. Capecitabine increases the anti-cancer activities while greatly reducing the toxic and side effects, thus widely applied in clinical use.

The structural formula of the capecitabine is as follows:

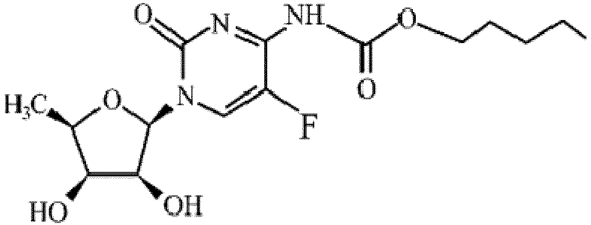

With respect to tegafur, capecitabine shows obvious difference in both molecular structure and in-vivo metabolic way. Due to intolerable side effects and limited effects, tegafur is seldom clinically used alone. Capecitabine has become the first choice of the anti-metabolism drugs for clinical cancer treatment in virtue of excellent targeting capability and relatively low side effects thereof. The comparison among 5-fluorouracil (5-FU), tegafur (FT-207) and capecitabine can be seen in table 1.

**Table 1**

| | | 5-Fluorouracil (5-FU) | Tegafur (FT-207) | Capecitabine |
|---|---|---|---|---|
| Identical points | | All the three drugs are 5-fluorouracil drugs, belong to anti-metabolism drugs, and mainly work in the S stage. The drugs are converted into 5-fluorouracil in vivo to take effect, and their anti-cancer spectra are similar. | | |
| Different points | Action mechanism | The drugs are converted by enzymes into 5--fluorodeouridine monophosphate and then can inhibit thymidine phosphorylase to synthesize enzymes and thus inhibit the synthesis of DNA. | The drugs are converted into 5-fluorouracil in vivo to take effect. The toxicity is 1/4-1/7 that of 5-fluorouracil, and the chemotherapeutic index is twice that of fluorouracil. | The drugs are converted into 5'-DFCR by carboxylesterase in the liver, then converted into 5'-DFUR by the cytidine deaminase of the liver and the cancer tissues, and finally catalyzed by the thymidine phosphorylase in the cancer tissues and converted into 5-FU to take effect. (Targeting) |
| | Pharmacokinetic characteristics | Incomplete oral absorption t_{1/2}10∼ 20min, mainly metabolized in the liver, finally decomposed into α-fluoro-β-alanine, ammonia, urea, and CO₂, the majority of which are discharged via the respiratory tract. | Good oral absorption t_{1/2}5h. Within 24h after drug delivery, 23% is discharged in the original form along with the urine, and 55% is discharged in the form of CO₂ via the respiratory tract. It has higher lipid solubility and can pass the blood brain barrier. | Quickly absorbed by intestinal mucosa after being taken orally. t_{1/2} 0.5 ∼ 1.0h. The majority of the metabolites are discharged along with the urine. The 5-FU concentration in the cancer tissues is 100 times higher than that in the blood after the drug is taken orally. |
| | Characteristics | Large side effects, short half-life period, no targeting capability. | Large side effects, long half-life period, no targeting capability. | Small side effects, long half-life period, targeting capability. |

### 3. TS-1:

Trademark: TS-1, manufactured by Taiho Pharmaceutical Co., Ltd. TS-1 is a three-ingredient anti-cancer drug containing tegafur, Gimeracil, and oxonic acid or salts thereof that serve as the active ingredients.

Gimeracil (CDHP) and Oteracil potassium (OXO) have no obvious anti-cancer activities when individually used, and can be used in combination with Tegafur to improve the treatment effect and reduce toxicity.

The CDHP plays the role of improving the anti-cancer effects of the Tegafur (FT-207). After being taken orally and entering the human body, FT-207 is converted into 5-FU by the catalyst effect of the kinase P450 of the liver first; then, 10% of the 5-FU enters the intestinal tract, and performs phosphorylation via the catalysis effect of the orotate phosphoribosyl transferase (ORTC), and then, catalyzed by dihydropyrimidine dehydrogenase (DPD) of the liver, roughly 90% of the remaining 5-FU follows the metabolic pathways of the 5-FU and is converted into two active products, namely fluoroside triphosphate (FUTP) and doxifluridine monoethyl-phosphate (FdUMP), which play the anti-cancer role. Therefore, DPD is the main rate-limiting enzyme for 5-FU degradation, and the maintaining of the plasma 5-FU level depends on the activities of the DPD. The CDHP is a reversible inhibitor of the DPD, has a DPD inhibiting effect which is 180 times that of uracil, and therefore can effectively inhibit the degradation of the 5-FU.

The OXO mainly plays the role of inhibiting the activities of the ORTC of the small intestine tissues. During the metabolic process of the tegafur, about 10% of 5-FU enters the intestinal structure, and performs phosphorylation via the catalysis effect of the orotate phosphoribosyl transferase. This process is deemed as the main factor of generating toxic and side effects in the intestinal tract. The OXO has an action characteristic of effectively inhibiting the ORTC and then inhibiting the phosphorylation of the 5-FU. The OXO has another obvious characteristic that after entering the human body via the mouth, the majority of the OXO is distributed on the surfaces of the intestinal mucosa cells, and only a very little enters the blood circulation, cancer tissues and other normal tissues.

Due to the synergy of the CDHP, the anti-cancer effect of the anti-cancer drug is improved. The 5-FU degradation inhibition effect of the CDHP is 180 times that of the uracil. Moreover, when the tegafur and the CDHP are used in combination, the OXO and the salts thereof particularly inhibit the possible increase of the toxicity in the intestines and stomach along with the improvement on the anti-cancer effect, thus reducing the side effects.

### 4. DPD inhibitor + 5-FU prodrug combination

The 5-FU must be converted into pseudouridine nucleotide and pseudodeoxyuridine nucleotide through metabolism to be activated. The pseudouridine nucleotide can interfere with DNA synthesis and RNA functions. The 5-FU and the natural counterpart thereof, namely uracil, are only different in the fluoro-substitution at position 5, so 5-FU is easily activated in the body of a cancer patient. However, the structural similarity of the 5-FU to the uracil also results in the 5-FU being quickly and widely converted into degradation products without anti-cancer activities and becoming inactivated. Dihydropyrimidine dehydrogenase (DPD) is the first and limiting step of the 5-FU degradation (inactivation). Research show that the half-life period of the 5-FU in plasma can be prolonged by inhibiting the DPD. At present, a plurality of DPD inhibitors have been researched, including inhibitors that inactivate the DPD irreversibly, and inhibitors that inactivate the DPD reversibly. 5-Ethynyluracil, also called 5-Ethynyluracil, is an irreversible inactivator of DPD, which can reduce or eliminate the metabolic inactivation of the 5-FU, so that with the existence of an extremely small amount of 5-Ethynyluracil, DPD is damaged and cannot inactivate the 5-FU. For this reason, the patent CN101068549 discloses the combined use of the DPD inhibitor (5-Ethynyluracil) and the 5-FU prodrug (capecitabine) to resist cancers.

EP2716290A1 discloses an anti-cancer combination comprising (a) tegafur, (b) gimeracil, and (c) oteracil potassium.

### BRIEF SUMMARY OF THE INVENTION

The first objective of the invention is to provide a cancer-treating pharmaceutical composition. In particular, the invention provides a pharmaceutical composition which can strengthen the anti-cancer effect of the capecitabine, and reduce the side effects.

The second objective of the invention is to provide a pharmaceutical kit for clinical use which can strengthen the anti-cancer effect of the capecitabine and reduce the side effects.

The third objective of the invention is to provide a therapy for the susceptibilities of the mammals to 5-fluorouracil.

According to the above mentioned current situations, the inventor had performed comprehensive research, and studied a pharmaceutical composition for strengthening the anti-cancer effects of capecitabine in conjunction with the action mechanism and effects of the TS-1. The results show that the anti-cancer effects of capecitabine can be obviously increased on the basis of reduction of the side effects when an ineffective-dose capecitabine that does not take effect under the condition of being used alone is used in combination with an effective dose of CDHP with anti-cancer synergy and an effective dose of OXO capable of reducing side effects.

The invention provides a pharmaceutical composition for cancer treatment, and the capecitabine, Gimeracil, and Oteracil potassium in the pharmaceutical composition are in a molar ratio of 1:0.1-3:0.5-4.

As an optimization, the Capecitabine, Gimeracil, and Oteracil potassium in the pharmaceutical composition are in a molar ratio of 1:0.4:1.

The three ingredients in the invention all are known compounds, all of which can be prepared by a conventional method.

Gimeracil includes pharmacologically acceptable salts thereof. In the invention, the chloridions of the Gimeracil can be replaced by halide ions, for example, fluorine atoms, bromine atoms, iodine atoms, etc., preferably 2,4-dyhydroxy-5-chloro pyridine, and 2,4-dyhydroxy-5-cyanopyridine.

The Oteracil potassium includes pharmacologically acceptable salts thereof, which include acid-addition salts and salts of alkali compounds. Useful acids, capable of generating acid-addition salts, include inorganic acids such as hydrochloric acid, sulfuric acid, phosphoric acid, hydrobromic acid, etc., and oxalic acid, succinic acid, maleic acid, fumaric acid, malic acid, tartaric acid, citric acid, malonic acid, methanesulfonic acid, benzoic acid and similar organic acids. Useful alkali compounds, capable of generating pharmacologically acceptable salts of alkali compounds, include sodium hydroxide, potassium hydroxide, calcium hydroxide, sodium carbonate, potassium bicarbonate, etc.

It should be particularly explained that, the key technical characteristic of the invention lies in the discovery that the combined use among the Gimeracil, Oteracil potassium and Capecitabine can strengthen the anti-cancer effect and reduce side effects, while the molar ratio of the three ingredients is not the key technical characteristic of the invention. The three ingredients are known compounds, and the doses thereof on the mammals fall within specific effective numerical ranges. Therefore, the invention is not limited to the above mentioned molar ratio ranges, but include all dose ranges which are safe to mammals.

The composition with the anti-cancer synergy of the invention is obtained through preparation of the compounds of the Gimeracil and Oteracil potassium or a single preparation thereof or two corresponding individual preparations. The single preparation or two corresponding individual preparations can be independently used or used in combination with an optional dosage form of capecitabine.

The invention also provides a pharmaceutical kit I for cancer treatment for strengthening the anti-cancer effects of the capecitabine and reducing the side effects of the capecitabine after combined use of the drugs. The kit I includes an ingredient (I) and an ingredient (II). The ingredient (I) is Gimeracil with anti-cancer synergy in an effective dose, and the ingredient (II) is Oteracil potassium with a side effect reduction effect in an effective dose.

Preferably, ingredient (I) and ingredient (II) are accommodated in separate containers. The two ingredients can be respectively mixed with pharmacologically acceptable carriers to be supplied to a preparation which can be applied in an optional dose unit.

Preferably, ingredient (I) and ingredient (II) are accommodated in the same container. The two ingredients can be mixed with pharmacologically acceptable carriers to be supplied to a preparation which can be applied in an optional dose unit.

The kit can deliver the drug at any time during the use of the capecitabine, which means that the kit can be used before, during, or after the use of the capecitabine.

The invention also provides a pharmaceutical kit II for cancer treatment. The kit II comprises three ingredients, respectively ingredient (I), ingredient (II) and ingredient (III), wherein the ingredient (I) is an effective dose of Capecitabine with an anti-cancer effect, ingredient (II) is an effective dose of Gimeracil with an anti-cancer synergy, and ingredient (III) is an effective dose of Oteracil potassium capable of reducing side effects.

Preferably, the three ingredients are respectively accommodated in different containers. The three ingredients (Capecitabine, Gimeracil and Oteracil potassium) all are known compounds, and can be mixed with pharmacologically acceptable carriers to be supplied to a preparation which can be applied in an optional dose unit.

Preferably, ingredient (I) is accommodated in an individual container, ingredient (II) and ingredient (III) are accommodated in the same container. Ingredient (II) and ingredient (III) can be mixed with pharmacologically acceptable carriers to be supplied to a preparation which can be applied in an optional dose unit.

Preferably, ingredient (II) and ingredient (III) are accommodated in different containers, and ingredient (I) is included in different containers that respectively contain ingredient (II) and ingredient (III). Ingredient (I) and ingredient (II) can be mixed with pharmacologically acceptable carriers to be supplied to a preparation which can be applied in an optional dose unit. Ingredient (I) and ingredient (III) can be mixed with pharmacologically acceptable carriers to be supplied to a single preparation which can be applied in an optional dose unit.

The three ingredients of kit II can be used at the same time, or used at any random combined sequence in no particular order.

The invention provides an anti-cancer drug for treating the susceptibilities of mammals to 5-fluorouracil diseases. The effective dose of capecitabine with an anti-cancer effect, the effective dose of Gimeracil with anti-cancer synergy, and the effective dose of Oteracil potassium capable of reducing side effects are applied to mammals.

In the above applications, the dose of the capecitabine is 0.05-800mg/kg, the dose of the Gimeracil is 0.05-400mg/kg, and the dose of the Oteracil potassium is 0.05-800mg/kg.

### BRIEF DESCRIPTION OF THE SEVERAL VIEWS OF THE DRAWINGS

FIG.1 is a histogram of cancer weights two weeks after delivery of the solvent group, the Capecitabine group, and the Capecitabine + Gimeracil group in embodiment 1;
FIG.2 is a histogram of cancer volumes two weeks after delivery of the solvent group, the Capecitabine group, and the Capecitabine + Gimeracil group in embodiment 1;
FIG.3 is a line chart of cancer volumes two weeks after delivery of the solvent group, the Capecitabine group, and the Capecitabine + Gimeracil group in embodiment 1;
FIG.4 is a survival rate diagram of the solvent group, the Capecitabine group, the Capecitabine + Gimeracil group, and the Capecitabine + 5-Ethynyluracil group in embodiment 1;
FIG.5 is a line chart of the cancer volumes of the solvent group, the Capecitabine group, the Capecitabine + Gimeracil group, and the Capecitabine + 5-Ethynyluracil group in embodiment 1;
FIG.6 is a survival rate diagram of the solvent group, the 5-FU + Gimeracil group, and the Capecitabine + Gimeracil group in embodiment 1;
FIG.7 is a line chart of cancer volumes of the solvent group, the capecitabine + Gimeracil group, and the Capecitabine + Gimeracil + Oteracil potassium group in embodiment 1;
FIG.8 is a line chart of weights of the solvent group, the Capecitabine + Gimeracil group, and the Capecitabine + Gimeracil + Oteracil potassium group in embodiment 1;
FIG.9 is a survival rate diagram of the solvent group, the Capecitabine + Gimeracil + Oteracil potassium group, and the Tegafur + Gimeracil + Oteracil potassium group in embodiment 1; and
FIG.10 is a line chart of the weights of the solvent group, the Capecitabine + Gimeracil + Oteracil potassium group, and the Tegafur + Gimeracil + Oteracil potassium group in embodiment 1.

### DETAILED DESCRIPTION OF THE INVENTION

The invention is described in further detail in conjunction with the attached drawings. The following embodiments are merely used to more clearly describe the technical solutions of the invention, and cannot be regarded as limits in the protective scope of the invention.

### Embodiment 1:

### Experimental solution:

Nude mice were transplanted with colonic cancer cells HCT116; after the cancer volumes reached 200-300mm3, the mice were randomly grouped, 8 pieces in each group; the mice were fed with drugs for two weeks. The drug delivery solution can be seen in Table 2 below:

**Table 2**

| Group | Animal quantity | Drug delivery means | Dose mg/kg | Molar ratio | Drug volume ml/kg | | Concentration mg/ml |
|---|---|---|---|---|---|---|---|
| Solvent | 8 | ig | - | | 10 | - | |
| Capecitabine (low-level) | 8 | ig | 30 | 1 | 10 | | 3 |
| Capecitabine (low-level) + Gimeracil | 8 | ig | 30+4.86 | 1:0.4 | 10 | | 3+0.49 |
| Capecitabine (low-level)+ Gimeracil + Oteracil potassium | 8 | ig | 30+4.86+16.29 | 1:0.4:1 | 10 | | 3+0.49+1.63 |
| Capecitabine (low-level) + 5-Ethynyluracil | 8 | ig | 30+4.55 | 1:0.4 | 10 | | 3+0.45 |
| Tegafur + Gimeracil | 8 | ig | 16.7+4.86 | 1:0.4 | 10 | | 1.67+0.49 |
| Tegafur + Gimeracil + Oteracil potassium | 8 | ig | 16.7+4.86+16.29 | 1:0.4:1 | 10 | | 1.67+0.49+1.63 |
| 5-FU+ Gimeracil | 8 | ig | 10.86+4.86 | 1:0.4 | 10 | | 1.09+0.49 |
| Capecitabine (high-level) | 8 | ig | 60 | 1 | 10 | | 6 |
| Capecitabine (high-level) + Gimeracil | 8 | ig | 60+9.72 | 1:0.4 | 10 | | 6+0.98 |
| Capecitabine (high-level) + Gimeracil + Oteracil potassium | 8 | ig | 60+9.72+32.58 | 1:0.4:1 | 10 | | 6+0.98+3.26 |

Notes: The above mentioned ingredients include Capecitabine, Gimeracil, Oteracil potassium, 5-Ethynyluracil, Tegafur, and solvent: 0.5%CMCNa.

### Experimental data:

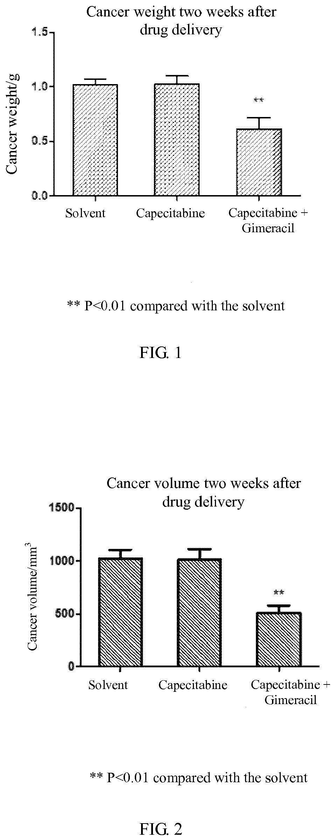

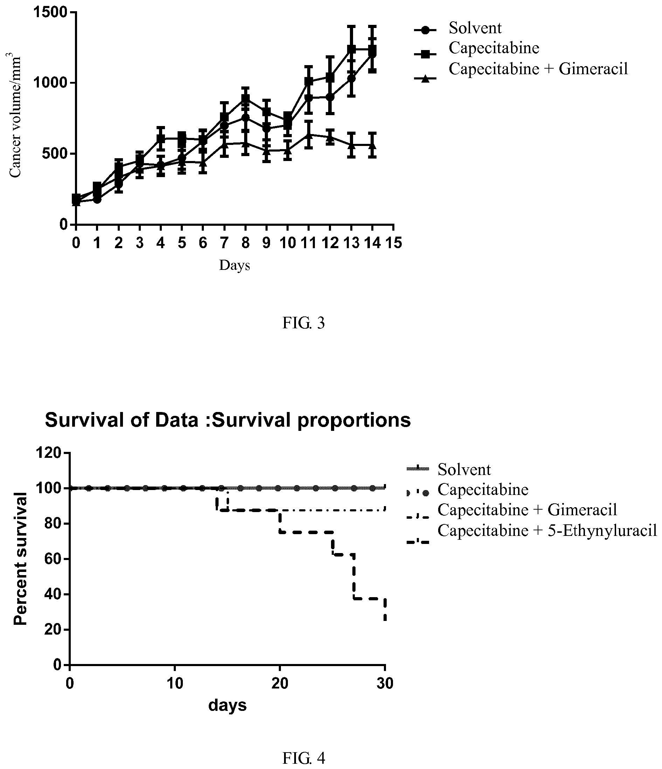

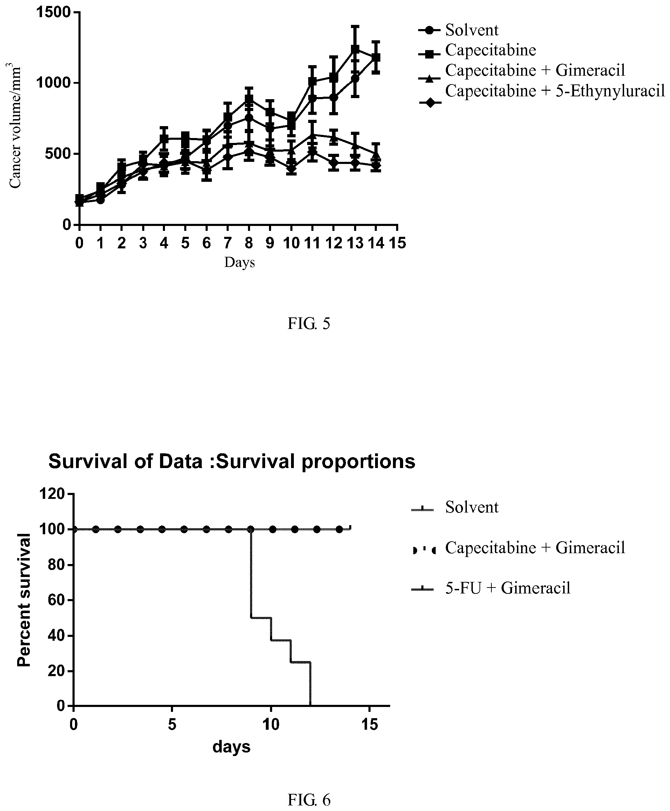

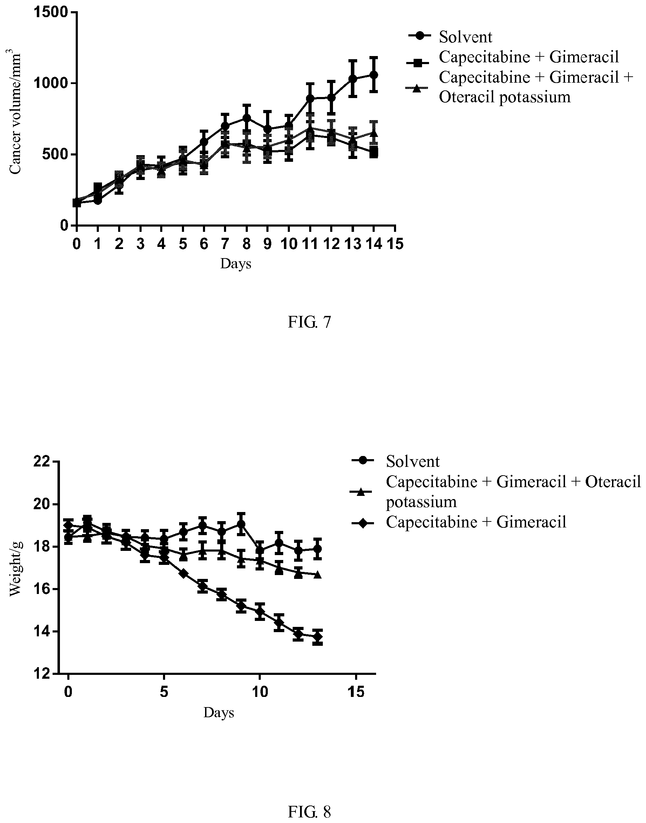

1. Purpose: To prove that Gimeracil has synergy with Capecitabine.
   From FIG.1, FIG.2, and FIG.3, it can be seen that the Capecitabine group in such dose failed to reduce the volume and weight of the cancer within 2 weeks, and has no statistical significance with respect to the solvent group. Compared with the Capecitabine group, the Gimeracil + Capecitabine group obviously reduced the volume and weight of the cancer, and has a statistical significance with respect to the solvent group.
2. Purpose: To prove that the synergism of the combination of the Gimeracil + Capecitabine is better than that of combination of the 5-Ethynyluracil + Capecitabine.
   From the cancer volume line chart in FIG.5, it can be seen that the treatment effects of the Capecitabine + Gimeracil group are equivalent to the treatment effects of the capecitabine + 5-Ethynyluracil group, but from the survival rate diagram in FIG.4 it can be seen that the toxic and side effects of the Capecitabine + 5-Ethynyluracil group are larger than those of the Capecitabine + Gimeracil group.
3. Purpose: To prove that the effect of the 5-FU + DPD inhibitor is inferior to that of the 5-FU prodrug + DPD inhibitor.
   The DPD inhibitor is Gimeracil. From the survival rate diagram in FIG.6, it can be seen that the toxic and side effects of the 5-FU + 5-Ethynyluracil group are larger than those of the capecitabine + Gimeracil group.
4. Purpose: To prove that the Oteracil potassium can reduce the side effects generated after the combined use of the Capecitabine + Gimeracil
   From the cancer volume in FIG.7, it can be seen that the treatment effects of the Capecitabine + Gimeracil + Oteracil potassium group are similar to those of the Capecitabine + Gimeracil group, but from the weight line chart in FIG.8, it can be seen that the weights are basically unchanged when the Capecitabine + Gimeracil + Oteracil potassium group is compared with those of the control group, and that the weights are greatly reduced when the Capecitabine + Gimeracil group is compared with the control group. Therefore, in comparison with the Capecitabine + Gimeracil group, the Capecitabine + Gimeracil + Oteracil potassium group can obviously reduce the side effects.
5. Purpose: To prove that the Capecitabine + Gimeracil + Oteracil potassium group is superior to TS-1

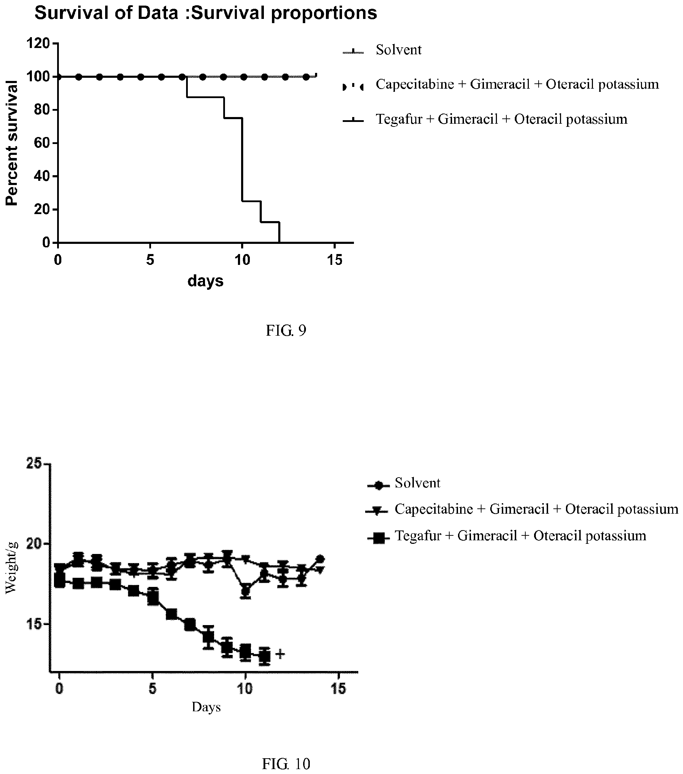

From the survival rate diagram in FIG.9, it can be seen that the survival rate of the TS-1 is obviously inferior to that of the Capecitabine + Gimeracil + Oteracil potassium group as time goes by. From the weight diagram in FIG.10, it can be seen that, obviously, there is no obvious difference between the weight of the Capecitabine + Gimeracil + Oteracil potassium group and the weight of the control group, while the weight of the TS-1 group is obviously reduced. Therefore, the Capecitabine + Gimeracil + Oteracil potassium group has smaller side effects in comparison with the TS-1 group.

### Embodiment 2:

### Experiment solution:

Nude mice were transplanted with colonic cancer cells HCT116; after the cancer volumes reached 100-200mm³, the mice were randomly grouped, 8 pieces in each group; the mice were fed with drugs for 19 days. The drug delivery solution can be seen in Table 3 below:

**Table 3**

| Group | Molar ratio | Drug delivery means | Animal quantity | Cancer volume (TV, mm³) | | RTV | Cancer weight(g) |
|---|---|---|---|---|---|---|---|
| | | | | dl | dl9 | | |
| Solvent control group | | ig | 8 | 117.11 ±8.02 | 3030.11 ±237.21 | 26.69 ±3.19 | 3.21±0.34 |
| Capecitabine + Gimeracil + Oteracil potassium 1 | 1:0.1:0.5 | ig | 8 | 118.89 ±7.01 | 2292.42 ±210.34 | 19.59 ±2.07 | 2.58±0.26 |
| Capecitabine + Gimeracil + Oteracil potassium 2 | 1:0.4:1 | ig | 8 | 118.46 ±6.43 | 1698.29 ±119.13 | 14.39 ±0.88** | 2.09±0.11* |
| Capecitabine + Gimeracil + Oteracil potassium 3 | 1:3:4 | ig | 8 | 118.57 ±7.85 | 1621.81 ±224.43 | 14.33 ±1.62** | 1.95±0.24* |

| | | | | | | | |
|---|---|---|---|---|---|---|---|
| Notes: Solvent control group: 0.5%CMCNa Capecitabine + Gimeracil + Oteracil potassium d1: The first day of drug delivery d19: end time of the drug delivery *p<0.05 **p<0.01, VS solvent control group. | | | | | | | |

### Experimental data:

From table 3 it can be seen that the three groups of compositions in different molar ratios all reduce the volumes and weights of the cancers within 19-day drug delivery time, and the Capecitabine + Gimeracil + Oteracil potassium groups 2, 3 have statistical significance with respect to the solvent control group.

## Claims

1. A pharmaceutical composition for cancer treatment, capable of strengthening the anti-cancer effect of Capecitabine and reducing side effects, **characterized in that** the pharmaceutical composition comprises an effective dose of Capecitabine with an anti-cancer effect, an effective dose of Gimeracil with an anti-cancer synergy, and an effective dose of Oteracil potassium for reducing side effects.

2. The pharmaceutical composition for cancer treatment according to Claim 1, **characterized in that** the Capecitabine, Gimeracil and Oteracil potassium in the pharmaceutical composition are in a molar ratio of 1:0.1-3:0.5-4.

3. The pharmaceutical composition for cancer treatment according to Claim 1, **characterized in that** the Capecitabine, Gimeracil and Oteracil potassium in the pharmaceutical composition are in a molar ratio of 1:0.4:1.

4. The pharmaceutical composition for cancer treatment according to Claim 1, **characterized in that** Gimeracil may include a pharmacologically acceptable salt thereof.

5. The pharmaceutical composition for cancer treatment according to Claim 1, **characterized in that** Oteracil potassium may include a pharmacologically acceptable salt thereof, such as an acid-addition salt, a salt of alkali compounds.

6. A pharmaceutical kit for cancer treatment, **characterized in that** the kit comprises three ingredients, respectively ingredient (I), ingredient (II), and ingredient (III), wherein the ingredient (I) is an effective dose of Capecitabine with an anti-cancer effect, ingredient (II) is an effective dose of Gimeracil with an anti-cancer synergy, and ingredient (III) is an effective dose of Oteracil potassium for reducing side effects.

7. The pharmaceutical kit for cancer treatment according to Claim 6, **characterized in that** Gimeracil may include a pharmacologically acceptable salt thereof.

8. The pharmaceutical kit for cancer treatment according to Claim 6, **characterized in that** Oteracil potassium may include a pharmacologically acceptable salt thereof, such as an acid-addition salt, a salt of alkali compounds.

9. The pharmaceutical composition according to any one of claims 1 to 5 or the pharmaceutical kit according to any one of claims 6 to 8, for use in treatment of cancer.

## Patentansprüche

1. Pharmazeutische Zusammensetzung zur Krebsbehandlung, die in der Lage ist, die Anti-Krebs-Wirkung von Capecitabin zu stärken und Nebenwirkungen zu verringern, **dadurch gekennzeichnet, dass** die pharmazeutische Zusammensetzung eine effektive Dosis von Capecitabin mit einer Anti-Krebs-Wirkung, eine effektive Dosis von Gimeracil mit einer Anti-Krebs-Synergie und eine effektive Dosis von Oteracil-Kalium zur Reduzierung von Nebenwirkungen aufweist.

2. Pharmazeutische Zusammensetzung zur Krebsbehandlung gemäß Anspruch 1, **dadurch gekennzeichnet, dass** das Capecitabin, das Gimeracil und das Oteracil-Kalium in der pharmazeutischen Zusammensetzung in einem Molverhältnis von 1:0,1-3:0,5-4 vorhanden sind.

3. Pharmazeutische Zusammensetzung zur Krebsbehandlung gemäß Anspruch 1, **dadurch gekennzeichnet, dass** das Capecitabin, das Gimeracil und das Oteracil-Kalium in der pharmazeutischen Zusammensetzung in einem Molverhältnis von 1:0,4:1 vorhanden sind.

4. Pharmazeutische Zusammensetzung zur Krebsbehandlung gemäß Anspruch 1, **dadurch gekennzeichnet, dass** Gimeracil ein pharmakologisch verträgliches Salz davon umfassen kann.

5. Pharmazeutische Zusammensetzung zur Krebsbehandlung gemäß Anspruch 1, **dadurch gekennzeichnet, dass** Oteracil-Kalium ein pharmakologisch verträgliches Salz davon umfassen kann, wie ein Säure-Zugabe-Salz, ein Salz von Alkaliverbindungen.

6. Pharmazeutisches Set zur Krebsbehandlung, **dadurch gekennzeichnet, dass** das Set drei Bestandteile aufweist, nämlich Bestandteil (I), Bestandteil (II) und Bestandteil (III), wobei der Bestandteil (I) eine effektive Dosis von Capecitabin mit einer Anti-Krebs-Wirkung, der Bestandteil (II) eine effektive Dosis von Gimeracil mit einer Anti-Krebs-Synergie und der Bestandteil (III) eine effektive Dosis von Oteracil-Kalium zur Reduzierung von Nebenwirkungen ist.

7. Pharmazeutisches Set zur Krebsbehandlung gemäß Anspruch 6, **dadurch gekennzeichnet, dass** Gimeracil ein pharmakologisch verträgliches Salz davon umfassen kann.

8. Pharmazeutisches Set zur Krebsbehandlung gemäß Anspruch 6, **dadurch gekennzeichnet, dass** Oteracil-Kalium ein pharmakologisch verträgliches Salz davon umfassen kann, wie ein Säure-Zugabe-Salz, ein Salz von Alkaliverbindungen.

9. Pharmazeutische Zusammensetzung gemäß irgendeinem der Ansprüche 1 bis 5 oder pharmazeutisches Set gemäß irgendeinem der Ansprüche 6 bis 8 zur Verwendung in der Krebsbehandlung.

## Revendications

1. Composition pharmaceutique pour le traitement du cancer, laquelle est capable de renforcer l'effet anti-cancer de Capecitabine et de réduire des effets secondaires, **caractérisée en ce que** la composition pharmaceutique comprend une dose effective de Capecitabine avec un effet anti-cancer, une dose effective de Gimeracil avec une synergie anti-cancer et une dose effective d'Oteracil potassium pour réduire des effets secondaires.

2. Composition pharmaceutique pour le traitement du cancer selon la revendication 1, **caractérisée en ce que** le Capecitabine, le Gimeracil et l'Oteracil potassium sont présents dans la composition pharmaceutique dans un rapport molaire de 1 :0,1-3 :0,5-4.

3. Composition pharmaceutique pour le traitement du cancer selon la revendication 1, **caractérisée en ce que** le Capecitabine, le Gimeracil et l'Oteracil potassium sont présents dans la composition pharmaceutique dans un rapport molaire de 1 :0,4 :1.

4. Composition pharmaceutique pour le traitement du cancer selon la revendication 1, **caractérisée en ce que** le Gimeracil peut comprendre un sel pharmacologiquement acceptable de celui-ci.

5. Composition pharmaceutique pour le traitement du cancer selon la revendication 1, **caractérisée en ce que** l'Oteracil potassium peut comprendre un sel pharmacologiquement acceptable de celui-ci, tel qu'un sel d'addition d'acide, tel qu'un sel de composés alcalins.

6. Kit pharmaceutique pour le traitement du cancer, **caractérisé en ce que** le kit comprend trois ingrédients, à savoir l'ingrédient (I), l'ingrédient (II) et l'ingrédient (III), dans lequel l'ingrédient (I) est une dose effective de Capecitabine avec un effet anti-cancer, l'ingrédient (II) est une dose effective de Gimeracil avec une synergie anti-cancer et l'ingrédient (III) est une dose effective d'Oteracil potassium pour réduire des effets secondaires.

7. Kit pharmaceutique pour le traitement du cancer selon la revendication 6, **caractérisé en ce que** le Gimeracil peut comprendre un sel pharmacologiquement acceptable de celui-ci.

8. Kit pharmaceutique pour le traitement du cancer selon la revendication 6, **caractérisé en ce que** l'Oteracil potassium peut comprendre un sel pharmacologiquement acceptable de celui-ci, tel qu'un sel d'addition d'acide, tel qu'un sel de composés alcalins.

9. Composition pharmaceutique pour le traitement du cancer selon l'une quelconque des revendications 1 à 5 ou le kit pharmaceutique selon l'une quelconque des revendications 6 à 8 pour l'utilisation dans le traitement du cancer.
